# EUROPEAN PATENT SPECIFICATION

(11) **EP 1 575 459 B1**
(45) Date of publication and mention of the grant of the patent: **21.05.2008**
(21) Application number: 03786920.3
(22) Date of filing: 19.11.2003
(51) Int. Cl.: A61F 2/46, A61F 2/44

(54) **SYSTEMS FOR INTERBODY SPINAL STABILIZATION WITH EXPANDABLE DEVICES**
SYSTEM ZUR INTERVERTEBRALEN STABILISATION MIT HILFE VON EXPANDIERBAREN VORRICHTUNGEN
SYSTEMES POUR LA STABILISATION INTERVERTEBRALE AU MOYEN DE DISPOSITIFS EXTENSIBLES

(30) Priority: 21.11.2002 US 428081 P
(43) Date of publication of application: 21.09.2005
(73) Proprietor: Warsaw Orthopedic, Inc., Warsaw, IN 46581 (US); Trieu, Hai H., Cordova, MN 38018 (US)
(72) Inventor: TRIEU, Hai, H., Cordova, MN 38018 (US)
(74) Representative: Hughes, Andrea Michelle
(86) International application number: PCT/US2003/037181
(87) International publication number: WO 2004/047691

(56) References cited:
- WO-A-00/35389
- WO-A-01/19295
- US-A- 6 127 597
- US-B1- 6 190 414

## Description

### BACKGROUND

With spinal deformities the disc space height between adjacent vertebrae of the spine can be lacking or abnormal due to the condition of the disc space or due to conditions created during surgery. Restoration of the disc space height during surgery can require insertion of instruments to provide and maintain distraction of the disc space during implant insertion. The use of such instruments requires time to accommodate such insertion and additional exposure of the operative site to accommodate the instruments.

Interbody fusion cages have been developed that provide the ability to adjust the height of the cage after insertion. Examples of such interbody fusion cages are disclosed in US 6,190,414 or WO 00/35389. However, such adjustment can require manipulation of cumbersome and intricate instruments in the cage to adjust the cage height. Such adjustment can also result in a non-uniform distribution of loads on the vertebral endplates at their interface with respective surfaces of the cage. Furthermore, internal expansion mechanisms in the cage reduce the space in the cage available for bone growth material.

There remains a need for spinal stabilization systems and methods that minimize the surgical exposure and number of instruments used during spinal surgery, reduce the time for insertion of stabilization devices, and reduce the potential for loss of stability.

### SUMMARY

Systems are provided for reducing the complexity and invasiveness of intervertebral spinal stabilization provide an expandable device deliverable to a spinal disc space with an expandable delivery instrument. The expandable devices are expanded in the disc space with an expandable element of the delivery instrument to distract the disc space and to implant the expanded device to provide stabilization.

According to one aspect, there is provided a system that includes a expandable device and a delivery instrument. The delivery instrument includes a distal expanding element conformable to a size and shape of an interior cavity of the device to apply an expansion force to the device after delivery of the collapsed expandable device to the surgical site with the delivery instrument.

According to one embodiment, the delivery instrument includes a distal expanding element in the form of a balloon expandable to apply an expansion force to the expandable device.

According to another embodiment, the expanding element is configured to apply an expansion force uniformly along the length of the expandable device to expand the expandable device in situ.

According to a further embodiment, the expandable device has an unexpanded configuration for deliver to the operative site in minimally invasive surgical procedures and is thereafter expandable with the delivery instrument to an expanded configuration for post-operative implantation at the surgical site.

Preferred embodiments will now be described, by way of example only, with reference to the drawings.

### BRIEF DESCRIPTION OF THE DRAWINGS

Fig. 1 is a sectional view of a collapsed expandable device and delivery instrument according to one embodiment.
Fig. 2 is the expandable device and delivery instrument of Fig. 1 in an expanded condition.
Figs. 3A and 3B are a plan view and an elevation view, respectively, of a spinal column segment having an intervertebral space prepared to receive a pair of expandable devices such as shown in Fig. 1.
Figs. 4A and 4B are a plan view and an elevation view, respectively, with the collapsed expandable device and delivery instrument of Fig. 1 in section and positioned in the prepared locations of the spinal column segment.
Figs. 5A and 5B are a plan view and an elevation view, respectively, with expandable devices and delivery instruments in section and expanded as shown in Fig. 2 in the prepared locations of the spinal column segment.
Figs. 6A and 6B are a plan view and an elevation view, respectively, with expanded expandable devices of Fig. 2 in section and in the prepared locations of the spinal column segment and the delivery instruments collapsed.
Figs. 7A and 7B are a plan view and an elevation view in partial section, respectively, with the expanded expandable devices of Fig. 2 in section and in the prepared locations of the spinal column segment and the delivery instruments removed.
Figs. 8A and 8B are a plan view and an elevation view, respectively, of a spinal column segment having an intervertebral space prepared to receive a pair of expandable devices according to another embodiment.
Figs. 9A and 9B are a plan view and an elevation view, respectively, with another embodiment collapsed expandable devices and delivery instruments in section and positioned in the prepared locations of the spinal column segment.
Figs. 10A and 10B are a plan view and an elevation view, respectively, with expanded expandable devices and delivery instruments in section and in the prepared locations of the spinal column segment.
Figs. 11A and 11B are a plan view and an elevation view, respectively, with expanded expandable devices in section and in the prepared locations of the spinal column segment and the delivery instruments collapsed.
Figs. 12A and 12B are a plan view and an elevation view, respectively, with the expanded expandable devices in section and in the prepared locations of the spinal column segment and the delivery instruments removed.
Fig. 13 is an end elevation view of the expandable devices of Figs. 7A and 7B in a spinal disc space.
Fig. 14 is an end elevation view of the expandable device of Figs. 12A and 12B in a spinal disc space.
Fig. 15 is an end elevation view of another embodiment pair of expandable devices in a spinal disc space.
Fig. 16 is a side elevation view of another embodiment expandable device in section in a spinal disc space.
Fig. 17 is a section view showing one approach to a spinal disc space for inserting one or more expandable devices.
Fig. 18 is a section view showing another approach to a spinal disc space for inserting one or more expandable devices.
Fig. 19 is a section view showing another approach to a spinal disc space for inserting one or more expandable devices.
Fig. 20 is a section view showing another approach to a spinal disc space for inserting one or more expandable devices.
Fig. 21 is an end view of another embodiment expandable device.
Fig. 22 is a perspective view of another embodiment expandable device in an unexpanded configuration.
Fig. 23 is an elevation view of another embodiment expandable device in an unexpanded configuration.
Fig. 24 is an elevation view of the expandable device of Fig. 23 in an expanded configuration.
Fig. 25 is an end view of the expanded expandable device of Fig. 24.
Fig. 26 is an elevational view of a deformed spinal column segment with an expandable device positioned in a disc space in an unexpanded condition.
Fig. 27 is the spinal column segment of Fig. 26 with the expandable implant expanded in the disc space.
Fig. 28 is the spinal column segment of Fig. 27 with a second expandable implant expanded in the disc space.
Fig. 29 is an elevational view of another embodiment expandable device in an unexpanded condition.
Fig. 30 is an elevational of the expandable device of Fig. 29 in an expanded condition.
Fig. 31 is an end view of the unexpanded expandable device of Fig. 29 with a distal portion of a delivery instrument therein.
Fig. 32 is the end view of the expandable device and delivery instrument portion of Fig. 31 with the distal portion of the delivery instrument and the expandable device expanded.
Fig. 33 is the end view of the expanded expandable device of Fig. 32 with an intermediate member positioned in a cavity thereof.
Fig. 34 is the end view of the expanded expandable device and intermediate member of Fig. 33 with the expandable device in a form which allows primary support to be provided by the intermediate member.
Figs. 35A and 35B are a plan view and an elevation view, respectively, of collapsed expandable devices and delivery instruments according to another embodiment in section and positioned in a collapsed disc space of the spinal column segment.
Figs. 36A and 36B are a plan view and an elevation view, respectively, of expanded expandable devices and delivery instruments in section and in a restored disc space of the spinal column segment.
Figs. 37A and 37B are a plan view and an elevation view, respectively, of expanded expandable devices in section and in the restored disc space of the spinal column segment and the delivery instruments removed.
Figs. 38A and 38B are a plan view and an elevation view, respectively, of collapsed expandable devices and delivery instruments according to another embodiment in section and positioned in a collapsed disc space of the spinal column segment.
Figs. 39A and 39B are a plan view and an elevation view, respectively, of expanded expandable devices and delivery instruments in section and in a restored disc space of the spinal column segment.
Figs. 40A and 40B are a plan view and an elevation view, respectively, of expanded expandable devices in section and in the restored disc space of the spinal column segment and the delivery instruments removed.
Figs. 41A and 41B are a plan view and an elevation view, respectively, of a collapsed expandable device and delivery instrument according to another embodiment in section and positioned in a collapsed disc space of the spinal column segment.
Figs. 42A and 42B are a plan view and an elevation view, respectively, of the expanded expandable device and delivery instrument in section and in a restored disc space of the spinal column segment.
Figs. 43A and 43B are a plan view and an elevation view, respectively, of the expanded expandable device in section and in the restored disc space of the spinal column segment and the delivery instrument removed.
Figs. 44A and 44B are a plan view and an elevation view, respectively, of a collapsed expandable device and delivery instrument in section and according to another embodiment positioned in a collapsed disc space of the spinal column segment.
Figs. 45A and 45B are a plan view and an elevation view, respectively, of the expanded expandable device and the delivery instrument in section and in a restored disc space of the spinal column segment.
Figs. 46A and 46B are a plan view and an elevation view, respectively, of the expanded expandable device in section and in the restored disc space of the spinal column segment and the delivery instrument removed.
Figs. 47A and 47B are elevational views of a delivery instrument with a distal expandable element in an unexpanded condition and expanded condition, respectively.
Figs. 48A and 48B are a plan view and an elevation view, respectively, of a collapsed expandable device and delivery instrument in section and according to another embodiment positioned in a collapsed disc space of the spinal column segment.
Figs. 49A and 49B are a plan view and an elevation view, respectively, of the expanded expandable device and the delivery instrument in section and in a restored disc space of the spinal column segment.
Figs. 50A and 50B are a plan view and an elevation view, respectively, of the expanded expandable device in section and in the restored disc space of the spinal column segment and the delivery instrument removed.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended. Any such alterations and further modifications in the illustrated devices, and any such further applications of the principles of the invention as illustrated herein are contemplated as would normally occur to one skilled in the art to which the invention relates.

There are described Systems and methods for positioning and deploying expandable devices in or between bony structures of a spinal column segment. Such systems can include instruments for delivering the expandable devices to the operative site and expanding the expandable devices in situ. Such expansion can distract adjacent vertebrae if positioned in an intervertebral space, restore deformed spinal column segments, and provide immediate and long-term support of one or more bony structures.

According to one embodiment, the delivery instrument includes a balloon catheter-type instrument having an expandable distal portion about which a collapsed expandable device is positioned and secured for delivery to the operative site. The delivery instrument can be employed in minimally invasive surgical procedures to deliver the collapsed or unexpanded expandable device to the operative site. Upon positioning the expandable device at the operative site, the distal portion of the delivery instrument is expandable to deploy and expand the expandable device at the operative site. Such deployment and expansion of the expandable device can, for example, distract adjacent vertebrae to provide a desired disc space height when positioned in an intervertebral space.

The systems of the present invention can be employed in minimally invasive surgical approaches to the spine. Such approaches include anterior, posterior, transforaminal, lateral, oblique, transpedicular and other approaches to the disc space. The approaches can be uni-portal or multi-portal in nature. The approaches can be to any portion of the spinal column segment, including the sacral, lumbar, thoracic, and cervical regions. Distraction of the disc space with the expansion of the expandable device eliminates requirements for positioning of a distraction device in the disc space to maintain disc space distraction prior to insertion of the expandable device. The systems can be employed with any viewing system to assist in monitoring placement of the expandable device in the disc space and the expansion of the device with the distraction instrument. Examples of suitable viewing systems include fluoroscopic, endoscopic, microscopic, CT scan, X-ray, and naked eye visualization systems.

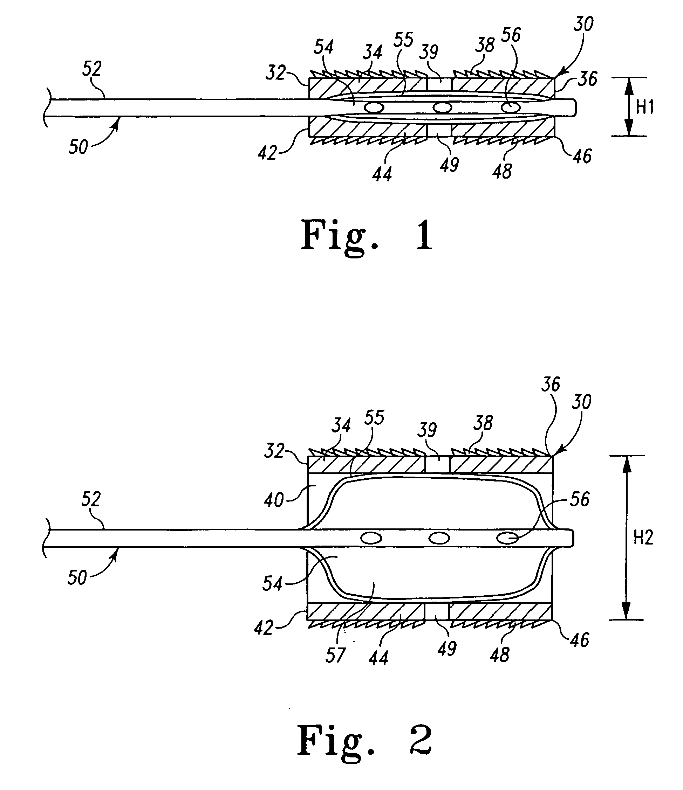

Referring now to Figs. 1 and 2, there is shown a first embodiment of an expandable device 30. In this embodiment, expandable device 30 includes an elongated body positionable in a spinal disc space that includes a first portion 34 positionable along one endplate of a first vertebra and a second portion 44 positionable along the endplate of an adjacent second vertebra. First portion 34 extends between a distal leading insertion end 36 and a proximal trailing end 32. Second portion 44 extends between a distal leading insertion end 46 and a proximal trailing end 42. A cavity 40 is defined between first portion 34 and second portion 44. Cavity 40 can extend between and open at distal end 36 and trailing end 32.

First portion 34 can be provided with a number of engagement members 38, and second portion 44 can also be provided with a number of engagement members 48. Engagement members 38, 48 are engageable with bony tissue of the vertebrae, and can be in the form of teeth, spikes, ridges, threads, barbs, knurlings, protrusions, fins, and combinations thereof, for example. It is further contemplated that the outer surfaces can be smooth, or auxiliary fixation or engagement members can be provided. First and second portions 34, 44 can further include one or more openings 39, 49, respectively, to facilitate bone ingrowth.

First portion 34 and second portion 44 are movable away from one another from an unexpanded configuration, as shown in Fig. 1, to an expanded configuration, as shown in Fig. 2. In the unexpanded configuration, expandable device 30 has a height H 1 between first portion 34 and second portion 44 as shown in Fig. 1. In the expanded configuration, expandable device 30 has a height H2 between first portion 34 and second portion 44. It is contemplated that height H 1 will allow expandable device 30 to be inserted, for example, in a disc space between adjacent vertebral bodies that is collapsed or otherwise deformed. Height H2 can correspond to a separation height between first and second portions 34, 44 required to provide a desired disc space height between adjacent vertebrae.

A delivery instrument 50 can be provided to move expandable device 30 from its unexpanded configuration to its expanded configuration. Delivery instrument 50 includes a proximal shaft 52 and a distal portion 54 including an expandable element 55. In the illustrated embodiment, expandable element 55 is an inflatable balloon-like structure having a collapsed configuration, as shown in Fig. 1, and an enlarged, inflated configuration, as shown in Fig. 2. Shaft 52 can be provided with a lumen through which fluid or material can be supplied through openings 56 to internal volume 57 of expandable element 55 to enlarge or inflate expandable element 55. Expandable element 55 is positionable in cavity 40 of expandable device 30 with each of the expandable element 55 and expandable device 30 in its unexpanded or collapsed configuration.

After delivery of expandable device 30 to the operative site, expandable element 55 can be inflated to provide an enlarged configuration for expandable element 55 and thus separate first and second portions 34, 44 of expandable device 30 as shown in Fig. 2. As expandable device 30 is expanded, first portion 34 and second portion 44 move away from one another and the volume of cavity 40 is increased. This expansion can distract adjacent vertebra to provide a desired spacing between the adjacent endplates and to restore a disc space height.

One example of a suitable delivery instrument 50 includes a high-pressure balloon catheter. Shaft 52 can be rigid, semi-rigid, or flexible. Shaft 52 can be fabricated from metals, polymers, or combinations thereof. Shaft 52 can be provided with at least one lumen to allow inflation or enlargement of expandable element 55 with a biocompatible fluid, such as air or saline, for example. Other embodiments contemplate that shaft 52 includes multiple lumens to, for example, deliver bone graft, bone growth material or other suitable filler material into the expanded cavity 40 of an expanded device 30. It is contemplated that expandable element 55 is collapsed prior to or simultaneously with placement of the filler material.

In the illustrated embodiment, distal portion 54 includes a single expandable element 55, although multiple expandable elements are also contemplated to provide distal portion 54 with alternate enlargement characteristics. For example, distal portion 54 could include a distal expandable element and a proximal expandable element having differing heights to provide angulation between the expanded first and second portions 34, 44 of expandable device 30. In another example, distal portion 54 can include an upper expandable element and a lower expandable element which can be selectively expanded move the adjacent one of first and second portions 34, 44 while the other of the first and second portions remains stationary. In a further example, expandable element 55 expands uni-directionally to move the adjacent one of the first and second portions 34, 44 in the direction of expansion.

In another embodiment, it is contemplated that distal portion 54 can be severed from shaft 52 after expansion, and post-operatively maintain expandable device 30 in an expanded condition. Accordingly, expandable element 55 can be inflated with bone growth material or other suitable filler material to facilitate bone growth or preserve motion of the intervertebral space through the expanded device 30. When the filler material suitably hardens in expandable element 55 to prevent flow from extending therefrom, shaft 52 can be removed. Alternatively or additionally, a valve arrangement can be provided adjacent expandable element 55 to prevent filler material from exiting therefrom. Expandable element 55 can be fabricated from porous material, resorbable material, or other suitable material to allow bone growth through the cavity of the expanded device. In a further embodiment, expandable element 55 is inflated with a polymer that is flowable into expandable element and thereafter polymerizes to form an elastic core between first and second portions 34, 44.

Expandable element 55 can include a size and shape that matches the size and shape of cavity 40 in its expanded configuration, although non-matching configurations are also contemplated. In the expanded configuration, expandable element 55 can apply a uniform expansion force along the inner wall surfaces of first portion 34 between leading end 36 and trailing end 32. If configured for bi-directional expansion as shown in Fig. 2, expandable element 55 can apply a uniform expansion force along second portion 44 between leading end 46 and trailing end 42. The uniform expansion force distributes the distraction loads along the adjacent vertebral endplate to provide uniform distraction along the length of expandable device 30. Expandable element 55 and/or cavity 40 can be provided with any suitable overall shape including conical, frusto-conical, spherical, cubic, spherical, polygonal, ovoid, long conical, long spherical, rectangular, tapered, stepped, dog-bone shape, offset shapes and combinations thereof.

Expandable element 55 can be made from any suitable material capable of withstanding the pressure supplied to enlarge or inflate expandable element 55 in situ. Examples include various polymeric materials, including polyethylene, terephthalates, polyolefins, polyurethanes, nylon, polyvinyl chloride, silicone or other suitable material. The material comprising expandable element 55 can be reinforced with woven or non-woven textile materials. Examples of suitable reinforcement materials include those that are polymeric and metallic in nature.

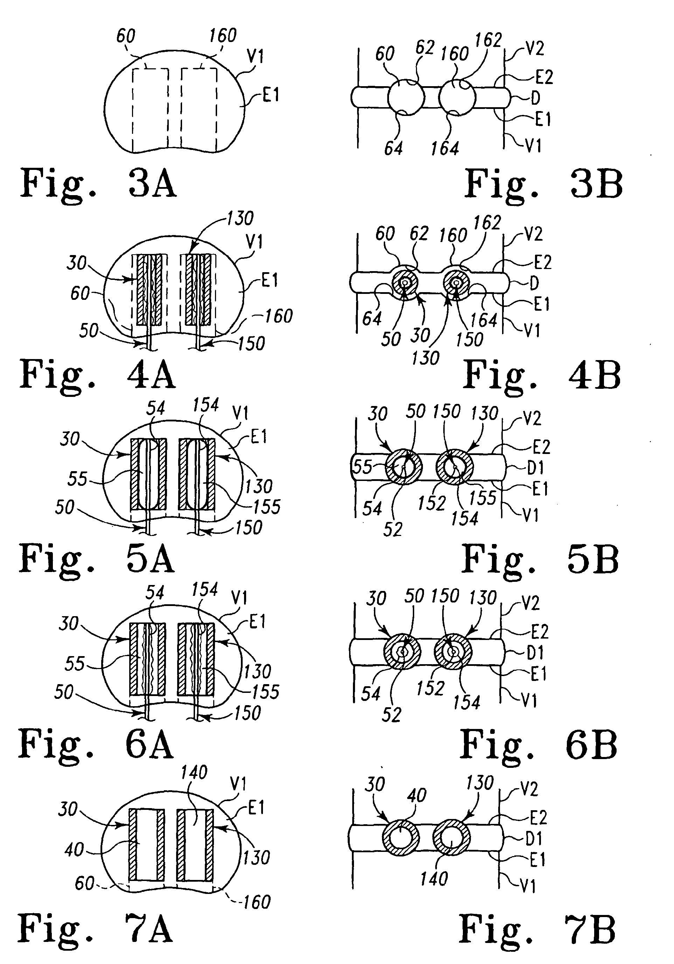

One example of a surgical technique employing expandable devices and delivery instruments in an intervertebral space will now be discussed with reference to Figs. 3A through 7B. Referring now to Figs. 3A and 3B, there is shown a spinal column segment including a lower vertebra V1 having an endplate E1, an upper vertebra V2 having an endplate E2, and an undistracted disc space D therebetween. After appropriate discectomy and endplate preparation, the undistracted disc space includes insertion locations 60, 160 for receiving expandable devices. Insertion location 60 includes a receiving bed 64 formed in endplate E1 and a receiving bed 62 formed in endplate E2 by any one or combination of reaming, scraping, cutting, or chiseling.

Receiving beds 62, 64 can be sized and shaped to match the outer surface profile of the portion of the expandable device to be positioned therein. Similarly, insertion location 160 can include a receiving bed 164 formed in endplate E1 and a receiving bed 162 formed in endplate E2, by any one or combination of reaming, scraping, cutting, or chiseling. Receiving beds 162, 164 can be sized and shaped to match the outer surface profile of the portion of the expandable device to be positioned therein. In the illustrated embodiment, receiving beds 62, 64, 162, 164 have a semi-circular cross-section to receive an expandable device having a circular or arcuate cross-section. Other shapes are also contemplated, including rectangular and square shaped cross-sections for the receiving beds. Still further it is contemplated that receiving beds are not formed, and the implants are placed into contact directly with the cortical bone of the endplates or with the endplates otherwise prepared.

As shown in Figs. 4A and 4B, unexpanded expandable devices 30, 130 are attached to collapsed expandable elements 55, 155 of delivery instruments 50, 150 for delivery to the operative site. Expandable devices 30, 130 are then placed into the prepared insertion locations 60, 160 in undistracted disc space D. A radio-contrast fluid, saline solution, compressed air, or other suitable fluid or substance can be delivered to expandable elements 55, 155 through a syringe or pump operable to provide sufficient pressure for distraction of the adjacent vertebrae. As the pressure and volume of the respective expandable elements 55, 155 increase, expandable devices 30, 130 are gradually expanded. The surfaces of the first and second portions of expandable devices 30, 130 come into contact with the prepared endplate locations 62, 64 and endplate locations 162, 164, respectively. Pressure in expandable elements 55, 155 is continually increased to inflate or enlarge expandable elements 55, 155 and expand expandable devices 30, 130 until the desired disc space D1 is achieved as shown in Figs. 5A and 5B. Accordingly, distraction of undistracted disc space D prior to insertion of the collapsed expandable devices 30, 130 is not necessary.

Expandable elements 55, 155 are then deflated or collapsed, as shown in Figs. 6A and 6B. Expandable elements 55, 155 can then be removed from their respective expanded devices 30, 130, as shown in Figs. 7A and 7B. Filler material can be deposited, packed, placed, delivered or injected into the cavities 40, 140 of expanded expandable devices 30, 130 and/or into the distracted disc space D1 to promote fusion and long term support of the adjacent vertebral bodies.

Any suitable osteogenic material or composition is contemplated for the filler material, including autograft, allograft, xenograft, demineralized bone, and synthetic and natural bone graft substitutes, such as bioceramics and polymers, and osteoinductive factors. The terms osteogenic material or osteogenic composition used herein broadly include any material that promotes bone growth or healing including autograft, allograft, xenograft, bone graft substitutes and natural, synthetic and recombinant proteins, hormones and the like.

Autograft can be harvested from locations such as the iliac crest using drills, gouges, curettes, and trephines and other tools and methods which are well known to surgeons in this field. Preferably, autograft is harvested from the iliac crest with a minimally invasive donor surgery. The osteogenic material may also include bone reamed away by the surgeon while preparing the endplates.

Natural and synthetic graft substitutes which replace the structure or function of bone are also contemplated for the osteogenic composition. Any such graft substitute is contemplated, including for example, demineralized bone matrix, demineralized bone matrix with bone chips, PMMA and other injectable synthetic bone cements, mineral compositions, and bioceramics. A vast array of bioceramic materials, including BIOGLASS®, hydroxyapatite, and calcium phosphate compositions known in the art which can be used to advantage for this purpose. Preferred calcium compositions include bioactive glasses, tricalcium phosphates, and hydroxyapatites. In one embodiment, the graft substitute is a biphasic calcium phosphate ceramic including tricalcium phosphate and hydroxyapatite.

In some embodiments, the osteogenic compositions used can comprise a therapeutically effective amount to stimulate or induce bone growth of a bone inductive or growth factor or protein in a pharmaceutically acceptable carrier. Osteoinductive factors that are recombinant human bone morphogenetic proteins (rhBMPs) are contemplated because they are readily available and do not contribute to the spread of infectious diseases. The bone morphogenetic protein can be a rhBMP-2, rhBMP-4 or heterodimers thereof. However, any bone morphogenetic protein is contemplated including bone morphogenetic proteins designated as BMP-1 through BPM-13.

The choice of carrier material for the osteogenic composition is based on biocompatibility, biodegradability, mechanical properties, and interface properties as well as the structure of the expandable device. Potential carriers include calcium sulphates, polylactic acids, polyanhydrides, collagen, calcium phosphates, polymeric acrylic esters, and demineralized bone. The carrier may be any suitable carrier capable of delivering the proteins. The carrier can be capable of being eventually resorbed into the body, such as an absorbable collagen sponge marketed by Integra LifeSciences Corporation under the trade name Helistat® Absorbable Collagen Hemostatic Agent. Another carrier is a biphasic calcium phosphate ceramic. Ceramic blocks are commercially available from Sofamor Danek Group, B.P. 4-62180 Rang-du-Fliers, France, and Bioland, 132 Rou d Espangne, 31100 Toulouse, France. The osteoinductive factor is introduced into the carrier in any suitable manner. For example, the carrier may be soaked in a solution containing the factor. One preferred embodiment contemplates use of OSTEOFIL® allograph paste sold by Regeneration Technologies, Inc. The allograph paste can be supplemented with a local autograft obtained from the cutting operation.

In the embodiment of Figs. 3A to 7B, expandable devices 30, 130 are radially expandable with delivery instruments 50, 150, respectively. It is contemplated that one of the expandable devices 30, 130 could be inserted into the appropriate disc space location, and then expanded to distract vertebrae V1 and V2. The other expandable device is then delivered to the other insertion location, and then expanded to at least contact the endplates of vertebrae V1 and V2. It is contemplated that the same delivery instrument 50 or 150 could be employed to deliver and expand each device. Alternatively, each expandable device 30, 130 could be pre-attached to separate delivery instruments 50, 150. Distraction of disc space D1 can be maintained with one of the delivery instruments 50, 150 in a delivered and expanded device 30, 130 while the other expandable device 30, 130 is delivered and expanded at the other disc space location.

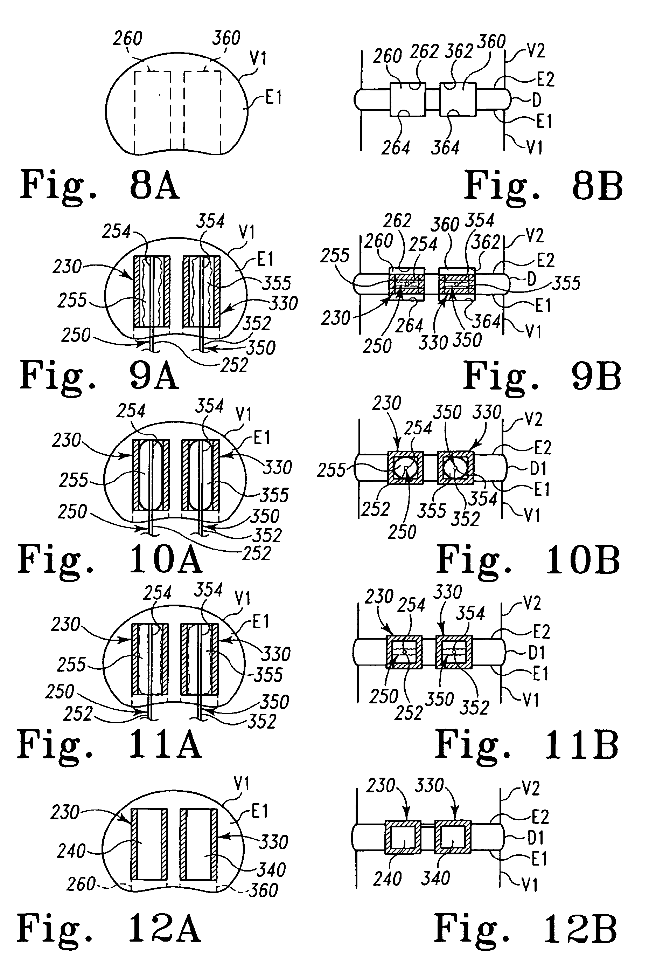

Referring now to Figs. 8A through 12B, there is shown another embodiment system and technique for delivering and expanding expandable devices in an intervertebral space. In Figs. 8A and 8B, there is shown a spinal column segment including lower vertebra V 1 having endplate E1, upper vertebra V2 having endplate E2, and undistracted disc space D therebetween. After appropriate discectomy and endplate preparation, the undistracted disc space includes insertion locations 260, 360 for receiving expandable devices. Insertion location 260 can include a receiving bed 264 formed in endplate E1 and a receiving bed 262 formed in endplate E2 by any one or combination of reaming, scraping, cutting, or chiseling the bone material from the endplates. Similarly, insertion location 360 can include a receiving bed 364 formed in endplate E1 and a receiving bed 362 formed in endplate E2 by any one or combination of reaming, scraping, cutting, or chiseling. Receiving beds 362, 364 can be sized and shaped to match the outer surface profile of the portion of the expandable device to be positioned therein. In the illustrated embodiment, receiving beds 262, 264, 362, 364 are formed with a rectangular cross-section to receive a correspondingly shaped portion of an expandable device positioned therein.

As shown in Figs. 9A and 9B, expandable devices 230, 330 are attached to collapsed expandable elements 255, 355 along distal portions 254, 354 of delivery instruments 250, 350, respectively. Expandable devices 230, 330 are then placed into the corresponding insertion locations 260, 360 in undistracted disc space D. A radio-contrast fluid, saline solution, compressed air, or other suitable fluid or substance can be delivered through shafts 252, 352 to expandable elements 255, 355 through a syringe or pump operable to provide sufficient pressure for expanding expandable devices 230, 330 and distract the adjacent vertebrae during such expansion. As the pressure and volume of the respective expandable elements 255, 355 increases, expandable devices 230, 330 are gradually expanded so that the first and second portions move away from one another toward the respective vertebrae V I and V2. Expandable devices 230, 330 are primarily expandable bi-directionally or uni-directionally in the vertical directions between the adjacent vertebrae.

The surfaces of the first and second portions of expandable device 230 come into contact with the prepared endplate locations 262, 264, and the surfaces of the first and second portions of expandable device 330 come into contact with endplate locations 362, 364. Expandable elements 255, 355 are enlarged or inflated until the desired disc space height D1 is achieved as shown in Figs. 10A and 10B. Accordingly, distraction of undistracted disc space D prior to insertion of the collapsed expandable devices 230, 330 is not necessary.

Expandable elements 255, 355 are then deflated or collapsed, as shown in Figs. 11A and 11B. Distal portions 254, 354 can then be removed from its respective expanded expandable device, as shown in Figs. 12A and 12B. Bone growth promoting material, including bone graft, bone graft substitutes, bone growth factors/carriers, allograft, autograft, therapeutic agents, and other suitable material can be deposited, packed, or injected into the cavities of expanded expandable devices 230, 330 and/or into the distracted disc space D1 to promote fusion and long term support of the adjacent vertebral bodies. In addition or alternatively, bone cement, including bioactive bone cements, could be deposited, packed or injected into the cavities of the expanded devices to provide long term support of the adjacent vertebrae.

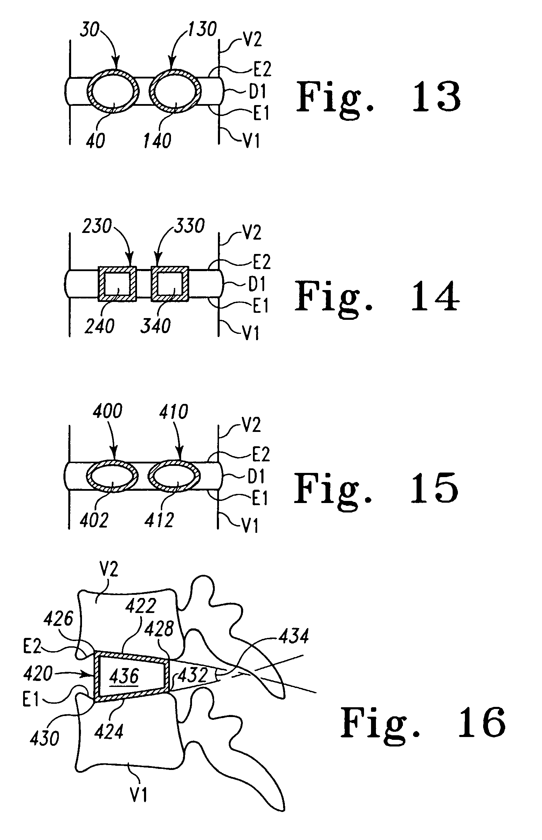

Various cross-sectional shapes and expansion characteristics for the expandable devices are contemplated. In Fig. 13 there is shown expandable devices 30, 130 in a spinal disc space having a circular cross-sectional shape. Expandable devices 30, 130 are radially expandable such that the height and lateral extent of each is increased upon expansion. In Fig. 14, there is shown expandable devices 230, 330 in a spinal disc space having a rectangular cross-sectional shape. Expandable devices 230, 330 are expandable bi-directionally or uni-directionally to vertically increase the height of each device while the lateral extent remains substantially the same. In Fig. 15, there is shown expandable devices 400, 410 in a spinal disc space having an oval cross-sectional shape. Expandable devices 400, 410 are primarily expandable bi-directionally or uni-directionally to vertically increase the height of each device while the lateral extent slightly expands or remains substantially the same. Other cross-section shapes are contemplated, including polygonal, elliptical, and racetrack shapes, for example.

It is further contemplated that more than two expandable devices could be positioned in an intervertebral space to restore the disc space height between vertebrae. It is also contemplated that a single expandable device could be positioned in an intervertebral space.

Various shapes along the length of the expandable devices are contemplated. For example, in Fig. 16 expandable device 420 is shown that includes a tapered cross-section along its length. Expandable device 420 includes a first portion 422 with posterior end 428 and anterior end 426. Expandable device 420 further includes a second portion 424 with posterior end 432 and an anterior end 430. In the expanded condition, as shown in Fig. 16, posterior ends 428, 432 are positioned closer to one another than anterior ends 426, 430. This positioning provides a taper between the upper and lower surfaces of expandable device 420 that restore the normal lordotic curvature of the spinal column segment when expandable device 420 is expanded with a correspondingly shaped distal portion of a delivery instrument in cavity 436. The relative positioning between the anterior and posterior ends could be reversed for normal kyphotic curvature.

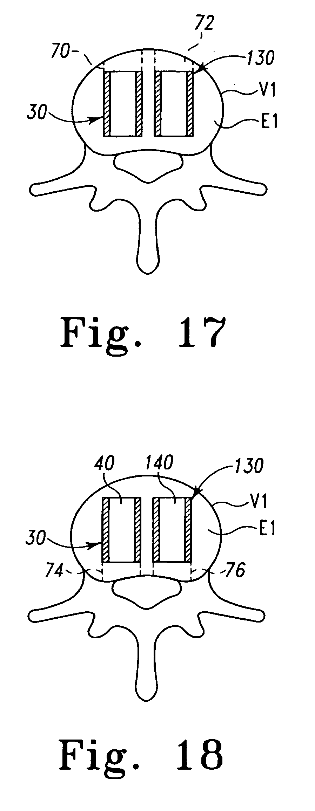

For intervertebral applications, various approaches to the spinal disc space are contemplated. In Fig. 17, an anterior approach used in which insertion locations 70, 72 are formed to receive a pair of side-by-side expandable devices 30, 130. Other embodiments contemplate an anterior approach in which a single expandable device is inserted that is sized and shaped to bi-laterally support the adjacent vertebrae.

In Fig. 18 a posterior approach is shown in which implant receiving locations 74, 76 are formed posteriorly in the disc space. Expandable devices 30, 130 are inserted into the implant receiving locations from a posterior approach in a collapsed condition, and thereafter expanded to distract the disc space. By inserting expandable devices 30, 130 in a collapsed state in a posterior approach, the amount of nerve root and muscle retraction in the approach to the disc space is minimized. Furthermore, there is no need for a separate distractor or other device in the disc space, which would occupy room in the disc space in order to maintain distraction during implant insertion. Such distractors can also occupy space in the anatomical space approaching the implant insertion location. Elimination of distractors provides additional space in the disc space for occupation by the expanded expandable device.

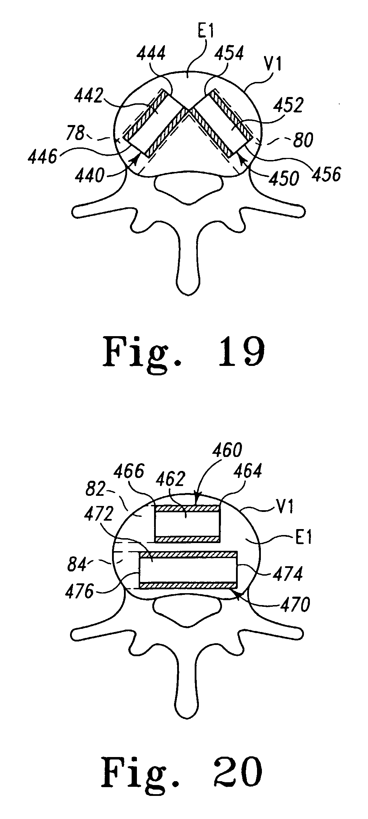

In Fig. 19 a posterior-lateral approach is shown in which insertion locations 78, 80 are formed obliquely in the disc space. Expandable devices 440, 450 are inserted into the implant receiving locations from a posterior lateral approach in a collapsed condition, and thereafter expanded to distract the disc space. Expandable devices 440, 450 form a V-shape in the disc space with the point of the V oriented anteriorly. By inserting expandable devices 440, 450 in a collapsed state in a posterior lateral approach, the approach is moved away from the spinal cord area along the posterior center of the vertebrae. Furthermore, there is no need for a separate distractor or other device to be positioned in the disc space during the procedure, providing additional space in the disc space for occupation by the expanded expandable device.

In Fig. 20 a lateral approach is shown in which insertion locations 82, 84 are formed and laterally spaced from one another across the disc space. Expandable devices 460, 470 are inserted into the insertion locations from a lateral approach in a collapsed condition, and thereafter expanded to distract the disc space. By inserting expandable devices 460, 470 in a collapsed state in a lateral approach, the amount of nerve root and muscle retraction can be minimized along with, in thoracic procedures, the spreading of the rib cage. Furthermore, there is no need for a separate distractor or other device to maintain distraction in the disc space which would occupy room in the disc space and in the approach to the disc space. Other embodiments contemplate a lateral approach in which a single expandable device is inserted that is sized and shaped to provide anterior and posterior support of the adjacent vertebrae.

In Fig. 20, anteriorly positioned expandable device 460 includes a body 462 extending between a leading insertion end 464 and an opposite trailing end 466. Posteriorly positioned expandable device 470 includes a body 472 extending between a leading insertion end 474 and an opposite trailing end 476. The length of body 462 can be less than the length of body 472 between the respective leading and trailing ends in order to accommodate the anterior positioning of expandable device 460 in the disc space.

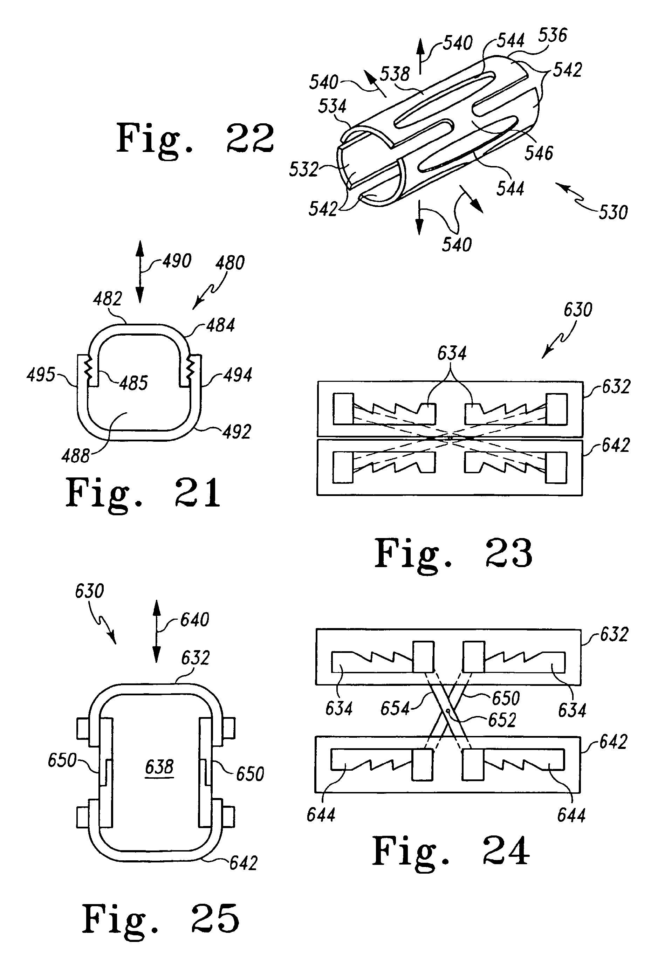

The expandable devices contemplated herein can be provided in various forms. For example, as shown in Fig. 21, the first and second portions of the expandable device could be adjustably connected along overlapping sidewalls of the first and second portions. Expandable device 480 includes a first portion 482 and a second portion 492 similar to expandable device 30 discussed above. First portion 482 includes opposite sidewalls 484, 485, and second portion 492 includes opposite sidewalls 494, 495. First portion 482 and second portion 492 define a cavity 488 therebetween into which expandable element 55 of delivery instrument 50 can be positioned.

The adjacent sidewalls 484, 494 include a number of interdigitating teeth that engage one another, and the adjacent sidewalls 485, 495 include a number of interdigitating teeth that engage one another. In the illustrated embodiment, the interdigitating teeth extend along all or a portion of the length of sidewalls 484, 485, 494, 495. The interdigitating teeth allow first and second portions 482, 492 to be uni-directionally or bi-directionally moved away from one another upon expansion of expandable element 55 in cavity 488 as indicated by arrows 490. The interdigitating teeth can include a ratcheted configuration that resists or prevents movement of first and second portions 482, 492 toward one another after expansion.

Expandable device 480 maintains support of the distracted vertebrae immediately after distraction, even after removal of expandable element 55 from cavity 488. The interdigitating teeth further define a number of expanded or separated positions between first and second portions 482, 492 that provide distraction heights that can be effected with a single expandable device 480. Accordingly, expandable device 480 is vertically collapsible to facilitate insertion in a collapsed disc space with the delivery instrument, and thereafter vertically expandable to distract the disc space and maintain distraction post-operatively.

In another example, the expandable devices could be made from a shape memory material or ductile material that is unexpanded or collapsed for positioning on the delivery instrument prior to insertion. Upon insertion in the spinal disc space, the device is radially expandable with inflation or enlargement of the delivery instrument to assume and maintain an expanded configuration. Expansion of the expandable device can be accomplished with temperature changes, chemical changes, or force induced changes with inflation or enlargement of the expandable element 55 of delivery instrument 50.

For example, in Fig. 22 there is shown expandable device 530 including a body 538 defining an interior cavity 532 extending between a proximal end 534 and a distal end 536. Body 538 includes a number of portions 542 therearound that each define an elongated flex opening 544. Adjacent segments 542 are interconnected by hinges 546. Body 538 is made of sufficiently ductile or formable material such that upon exertion of a radial expansion force, indicated by arrows 540, flex openings 544 can enlarge and hinges 546 can stretch to allow segments 542 to move away from one another, enlarging interior cavity 532 and distracting the adjacent vertebrae.

In a further example, the expandable devices can include a first mechanical configuration that allows a collapsed condition for insertion of the device with the delivery instrument. After insertion, the device can be mechanically adjusted upon inflation or enlargement of the distal portion of the delivery instrument to assume an expanded condition at the operative site. Examples of such expandable devices include those made from a wire mesh material, and devices with first and second portions connected by mechanical linkages, as shown in Figs. 23-25.

In Figs. 23-25 device 630 includes a first portion 632 and a second portion 642. Linkages 650 movably couple first and second portions 632, 642 to one another. Linkages 650 include first and second members 652, 654 pivotally coupled to one another. Members 652, 654 each include a first end positioned in respective ones of the receptacles 634 of first portion 632, and opposite second ends positioned in respective ones of the receptacles 644 of second portion 642. The ends of the members 650, 652 can include a configuration that interdigitates with a ratchet surface formed along the respective receptacles 634, 644. In the unexpanded configuration shown in Fig. 23, the ends of members 652, 654 are positioned at the outer ends of the respective receptacles 634, 644.

As first and second portions 632, 642 are bi-directionally moved away from one another with expandable element 55 in cavity 638, as indicated by arrows 640, the ends of members 650, 652 move longitudinally toward one another along the receptacles 634, 644 of each of the respective first and second portions 632, 642, as shown in Fig. 24. The rigid members 652, 654 move first and second portions 632, 642 away from one another, and engage the ratchet surfaces along receptacles 634, 644 to maintain the expanded or separated position between first and second portions 632, 642, as shown in Figs. 24 and 25. Accordingly, expandable device 630 is vertically collapsible to facilitate insertion in a collapsed disc space with the delivery instrument, and thereafter vertically expandable to distract the disc space and maintain distraction post-operatively.

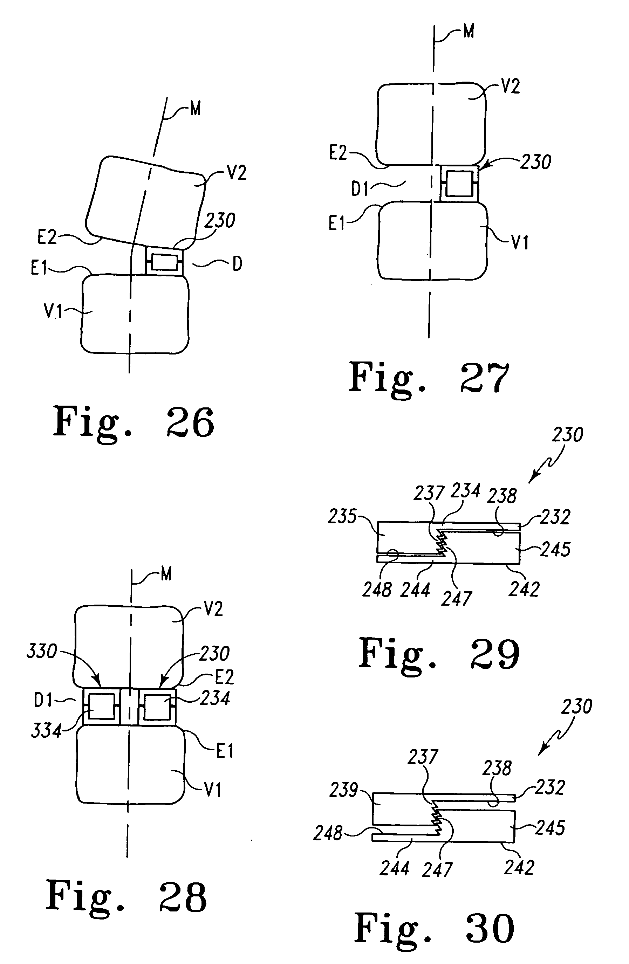

Referring now to Figs. 26-28, there is shown various steps of one technique for restoring a scoliotic spinal column segment with the expandable devices discussed herein. Figs. 26-28 will be discussed with reference to expandable devices 30, 330, it being understood that the other expandable device embodiments discussed herein would also have application in this technique. In Fig. 26 the spinal column segment includes vertebra V1 and vertebra V2 with deformed or collapsed disc D between endplates E1 and E2, respectively. Expandable device 230 is positioned in disc space D in an unexpanded condition on the side of spinal midline M to which the spinal column segment is concavely curved.

In Fig. 27, expandable device 230 is expanded with, for example, expandable element 255 of delivery instrument 250 to move the endplates E and E2 away from one another and into a more parallel or natural orientation, providing a restored disc space D1. In Fig. 28, a second expandable device 330 is positioned in disc space D1 on the opposite side of spinal midline M and expanded to provide balanced bi-lateral support of the spinal column segment.

Restoration of the desired spinal column segment with one or more additional expandable devices 230 positioned in the disc spaces of other vertebral levels is also contemplated for a multi-level scoliotic correction procedure. After expandable devices 230, 330 are expanded, the cavities can be filled with filler material, such as bone graft, bone graft substitutes, and bone growth promoting material for promoting fusion. If it is desired to maintain motion of one or more of the restored vertebral levels, then a motion preserving device can be inserted into the cavity of the expandable devices 230, 330, as discussed further below.

Figs. 29-30 provide an elevational view of an embodiment for expandable devices 230, 330. Expandable device 230 includes a first portion 232 and a second portion 242 which define cavity 240 therebetween. First portion 232 includes sidewalls 234 that each include an arm 235. A receptacle 238 is formed along one end of arm 235. Arm 235 includes engagement surfaces 23 extending along receptacle 238. Second portion 242 similarly includes sidewalls 244 that each include an arm 245 and a receptacle 248. Arm 245 is received in receptacle 238, and arm 235 is received in receptacle 248. Arm 245 includes engagement surfaces 247 extending therealong that are engageable with adjacent engaging surfaces 237 of arm 235 of first portion 232.

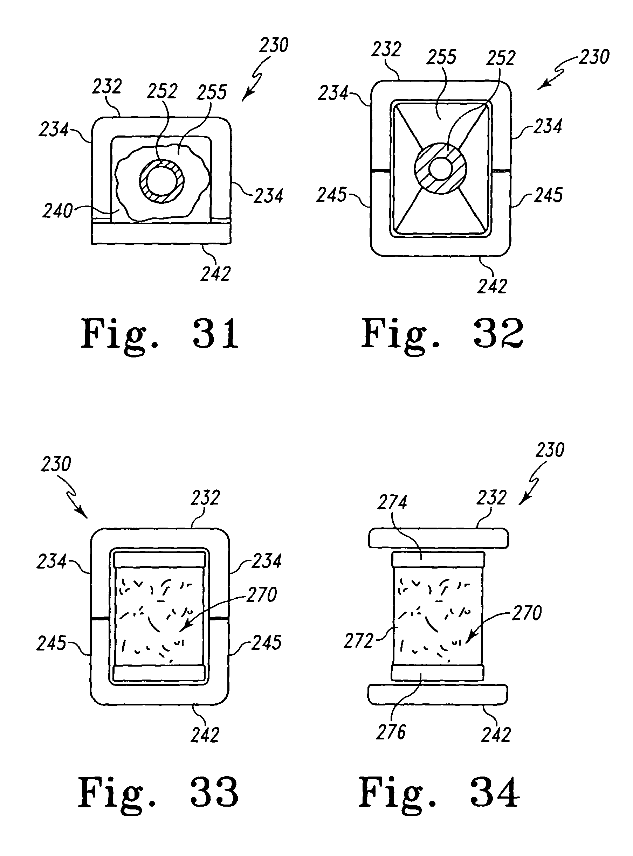

In Fig. 29 and 31, expandable device 230 is shown in a collapsed condition with arms 245 of second portion 240 fully recessed in the adjacent receptacles 238 of first portion 230. Expandable element 255 is positioned in cavity 240 in an unexpanded condition and expandable device 230 unexpanded. In Figs. 30 and 32, expandable element 255 is expanded to move first and second portion 232, 242 away from one another to an expanded configuration. The engagement surfaces 237, 247 can interdigitate and engage one another to maintain expandable device 230 in an expanded condition, even if compressive loads are applied to first and second portions 232, 242. The engagement surfaces 237, 247 can be provided with a ratcheted or other suitable configuration that allows movement of first and second portions 232, 242 away from one another but resists or prevents movement of first and second portion 232, 242 toward one another.

In Fig. 33, expandable element 255 is removed and a motion preserving device 270 is positioned in the expanded cavity 240 of expandable device 230. Motion preserving device 270 can include an elastic core 272 and upper and lower plates 274, 276 positionable along respective ones of the first and second portions 232, 242. With motion preserving device 270 positioned in cavity 240, contact between the engagement surfaces of first and second portions 232, 242 is eliminated so that the load is carried by elastic core 272. For example, one or both of the arms 235, 245 and/or engagement surfaces 237, 247 can be removed, or resorbed over time. Removal of the compressive load carrying capabilities between first and second portions 232, 242 allows the compressive load to be carried by elastic core 272.

Elastic core 272 allows motion between the adjacent vertebrae to be preserved while maintaining the desired positioning between the adjacent vertebral endplates. Transfer to elastic core 272 of the load carried between first and second portions 232, 242 by the rigid load-supporting arms 235, 245 can be accomplished by removing the load supporting arms and/or engagement surfaces 237, 247 between first and second portions 232, 242. In one embodiment, load removal can be accomplished by in vivo degradation of the rigid load supporting elements between first and second portions 232, 242. For example, the rigid load supporting arms 235, 245, or the entire first and second portions 232, 242, can be fabricated from bio-resorbable polymers or other bio-degradable or resorbable material.

In one embodiment, elastic core 272 can be formed in situ by injection of a polymerizable polymer into expandable element 255. The polymer causes expandable element 255 to expand and restore the disc space height by moving first and second portions 232, 242 away from one another and into contact with the adjacent vertebral endplates. After polymerization, shaft 252 can be detached and expandable element 255 remains in the cavity of expandable device 230. Expandable element 255 can be provided with a size and shape that conforms to the cavity between first and second portions 232, 242 to provide flexible load support along the entire length thereof. In embodiments employing this type of elastic core, expandable device 230 need not be provided with rigid load supporting arms between first and second portions 232, 242.

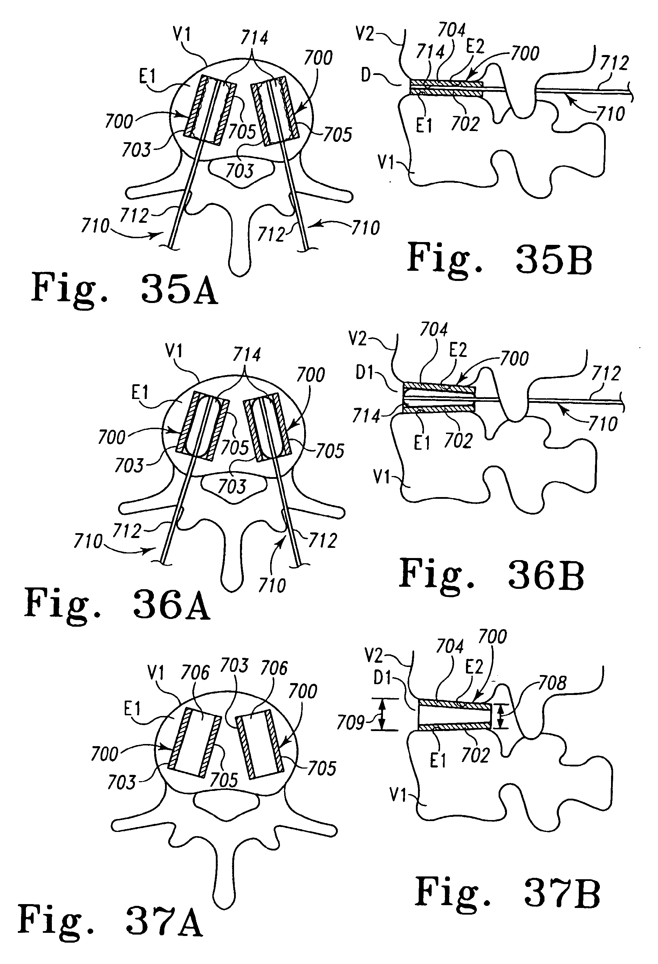

Figs. 35A and 35B are a plan view and an elevation view, respectively, of another embodiment pair of collapsed expandable devices 700 and associated delivery instruments 710 positioned in a collapsed disc space D between vertebrae V1 and V2. The collapsed or unexpanded expandable devices 700 are secured around the respective unexpanded expandable element 714 at a distal end of delivery instrument 710 for delivery to the collapsed disc space D. In the illustrated embodiment, disc space D is accessed from a posterior approach, although other approaches are also contemplated. Expandable device 700 includes a width between opposite sides 703, 705.

Figs. 36A and 36B are a plan view and an elevation view, respectively, with expandable devices 700 expanded with a fluid delivered through shafts 712 of delivery instruments 710. In the expanded condition, lower and upper surfaces 702, 704 of expandable device 700 act on the adjacent vertebral endplates E1, E2 to distract vertebrae V1, V2 and provide a restored disc space D1. The width between opposite sides 703, 705 of expandable devices 700 remains substantially constant during and after expansion. Accordingly, devices 700 are vertically expandable to increase their height while their widths remain constant.

Expandable devices 700 are tapered along the length thereof between an anterior end and a posterior end. In the illustrated embodiment, the posterior end includes a first height 708, and the anterior end includes a second height 709 which is greater than first height 708. This tapered height provides a desired angulation between the endplates E1, E2 as may be desired. It is also contemplated that device 700 can be tapered anteriorly.

Figs. 37A and 37B are a plan view and an elevation view, respectively, with the expanded expandable devices 700 in the restored disc space D1 of the spinal column segment and the delivery instruments 710 removed from cavities 706 of expandable devices 700. Filler material for fusion or maintenance of motion between the adjacent vertebrae can be placed in cavities 706.

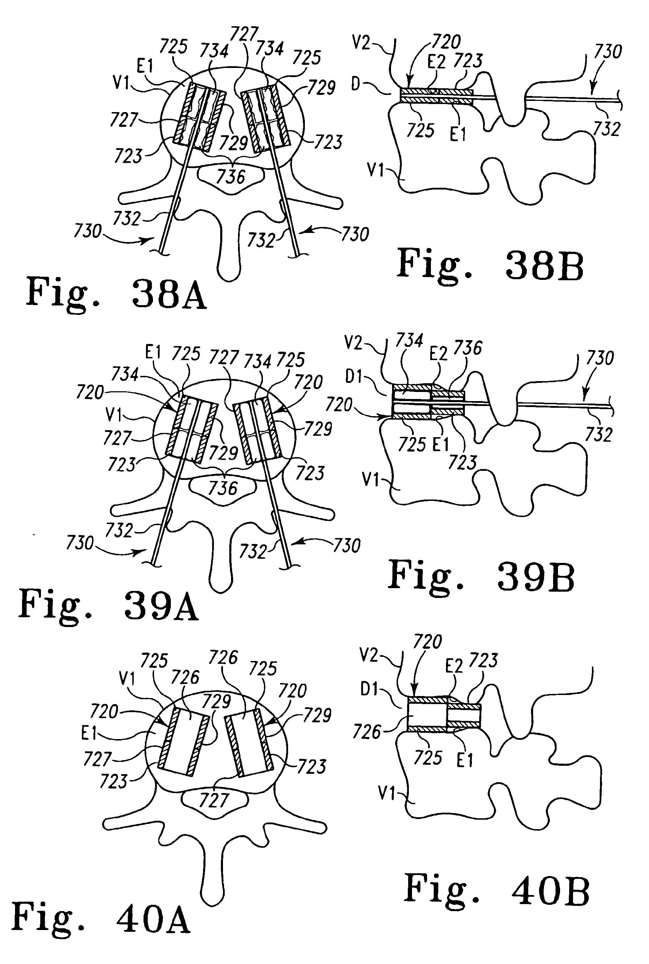

Figs. 38A and 38B are a plan view and an elevation view, respectively, of another embodiment pair of collapsed expandable devices 720 and associated delivery instruments 730 positioned in a collapsed disc space D between vertebrae V1 and V2. The collapsed or unexpanded expandable devices 720 are secured around the unexpanded expandable elements 734, 736 at a distal end of delivery instrument 730 for delivery to the collapsed disc space D. Expandable devices 720 each include a posterior portion 723 and an anterior portion 725, and a width between opposite sides 727, 729. The height and width of expandable device 720 is substantially uniform in its collapsed or unexpanded condition along portions 723, 725.

Figs. 39A and 39B are a plan view and an elevation view, respectively, with expandable devices 720 expanded with a fluid delivered through shafts 732 of delivery instruments 730. In the expanded condition, posterior and anterior portions 723, 725 of expandable devices 720 act on the adjacent vertebral endplates E1, E2 to distract vertebrae V1, V2 and provide a restored disc space D1. The width between opposite sides 727, 729 of expandable devices 720 remains substantially constant during and after expansion. Accordingly, expandable devices 720 are vertically expandable while the widths remain constant.

Expandable devices 720 are stepped in height between anterior portion 725 and posterior portion 723 to provide a greater anterior height for the expanded expandable devices 720. This stepped height provides a desired angulation between the endplates E1, E2. It is also contemplated that device 720 can be stepped down in height anteriorly. To facilitate this stepped distraction, delivery instrument 730 can be provided with an anterior expandable element 734 and a posterior expandable element 736. Expandable elements 734, 736 can be provided with differing heights in their expanded configurations that conform to the expanded height of respective ones of the anterior and posterior portions 725, 723 in which expandable elements 734, 736 are positioned.

Figs. 40A and 40B are a plan view and an elevation view, respectively, with the expanded expandable devices 720 in the restored disc space D1 of the spinal column segment and the delivery instruments 730 removed from cavities 726 of expandable devices 720. Filler material for fusion or maintenance of motion between the adjacent vertebrae can be placed in cavities 726.

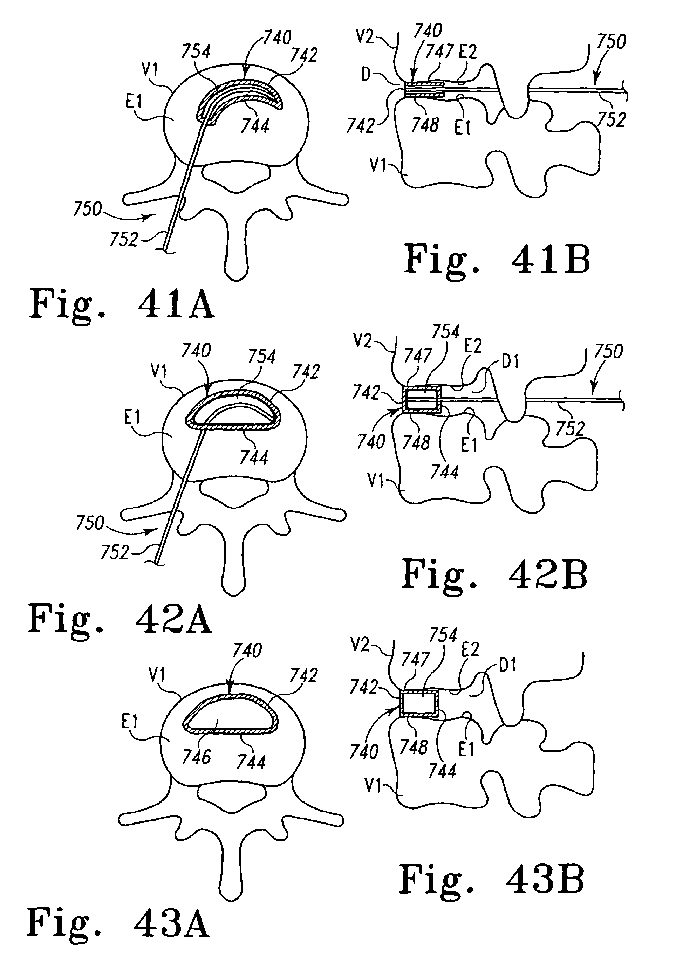

Figs. 41A and 41B are a plan view and an elevation view, respectively, of another embodiment collapsed expandable device 740 and associated delivery instrument 750 positioned in a collapsed disc space D between vertebrae V1 and V2. The collapsed or unexpanded expandable device 740 is secured around the unexpanded expandable element 754 at a distal end of delivery instrument 750 for delivery to the collapsed disc space D. Expandable device 740 includes, in the collapsed condition, a convexly curved anterior wall 742 and a concavely curved posterior wall 744. Walls 742, 744 form a banana or kidney shape that facilitates placement of expandable device 740 in the disc space for bi-lateral support of vertebrae V and V2 from a single approach.

Figs. 42A and 42B are a plan view and an elevation view, respectively, with expandable device 740 expanded with a fluid delivered through shaft 752 of delivery instrument 750. In the expanded condition, upper and lower portions 747, 748 of expandable device 740 act on the adjacent vertebral endplates E1, E2 to distract vertebrae V1, V2 and provide a restored disc space D1. Expansion of expandable device 740 can result in posterior wall 744 moving posteriorly such that in the expanded condition, posterior wall 744 is substantially linear to provide expandable device 740 with a D shape.

Expandable device 740 include convexly curved anterior wall 742 which facilitates placement of expandable device 740 along a curved insertion path in which the anterior wall 742 conforms to the profile of the curved anterior portion of endplates E1, E2. In the illustrated embodiment, expandable device 740 is positioned in the anterior half of disc space D. Expandable element 754 can be provided with a shape that conforms to the D-shaped interior cavity 746 when expanded.

Figs. 43A and 43B are a plan view and an elevation view, respectively, with the expanded expandable device 740 in the restored disc space D1 of the spinal column segment and the delivery instrument 750 removed from cavity 746 of expandable device 740. Filler material for fusion or maintenance of motion between the adjacent vertebrae can be placed in cavity 746.

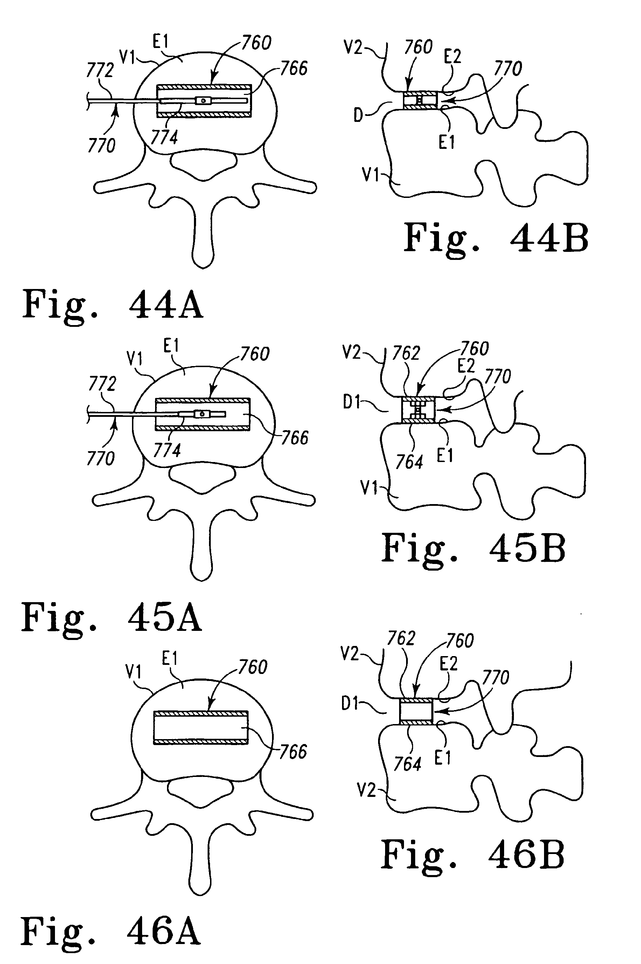

Figs. 44A and 44B are a plan view and an elevation view, respectively, of another embodiment collapsed expandable device 760 and associated delivery instrument 770 positioned in a collapsed disc space D between vertebrae V1 and V2. The collapsed or unexpanded expandable device 760 is secured around the unexpanded expandable element 774 at a distal end of delivery instrument 770 for delivery to the collapsed disc space D in a lateral approach. Expandable device 760 includes a first portion 762 and a second portion 764 engaged along opposite sides of expandable element 774 and positionable adjacent respective ones of the endplates E1 and E2.

Figs. 45A and 45B are a plan view and an elevation view, respectively, with expandable device 760 expanded by manipulating shaft 772 of delivery instrument 770 to expand expandable element 774. In the expanded condition, first and second portions 762, 764 of expandable device 760 act on the adjacent vertebral endplates E1, E2 to distract vertebrae V1, V2 and provide a restored disc space D1.

Figs. 46A and 46B are a plan view and an elevation view, respectively, with the expanded expandable device 760 in the restored disc space D1 of the spinal column segment and the delivery instrument 770 removed from cavity 766 of expandable device 760. Filler material for fusion or maintenance of motion between the adjacent vertebrae can be placed in cavity 766.

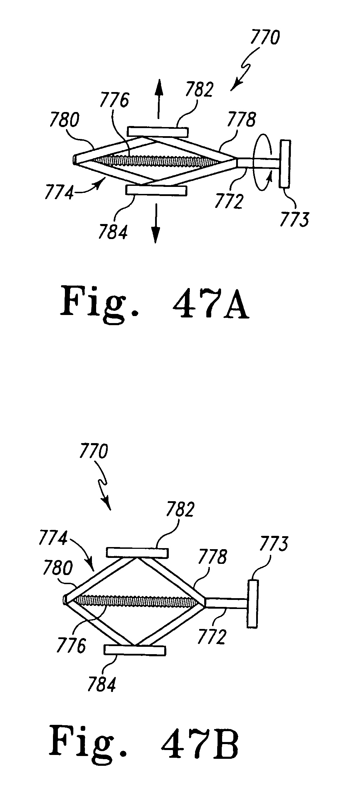

Further details of delivery instrument 770 are provided in Figs. 47A and 47B. Shaft 772 includes a proximal handle portion 773 and a distal portion 776 extending through expandable element 774. Expandable element 774 includes a first pivoting linkage 778 and a second pivoting linkage 780. Linkages 778, 780 each include an intermediate pivot point engaged to and movable with distal portion 776. Linkages 778, 780 further include distraction members 782, 784 coupled at the upper and lower ends thereof.

Distal portion 776 is coupled to linkages 778, 780 so that, as shaft 772 is rotated about its axis with handle portion 773 as indicated in Fig. 47A, the pivoting intermediate portions of linkages 778, 780 are drawn toward one another to move distraction members 782, 784 away from one another, as shown in Fig. 47B. When positioned in a cavity of an expandable device, distraction members 782, 784 contact adjacent portions of the expandable device to expand the expandable device and distract the disc space. When the desired distraction has been achieved, expandable device 770 can be removed from the implant by rotating shaft 772 in the opposite direction and move distraction members 782, 784 toward one another.

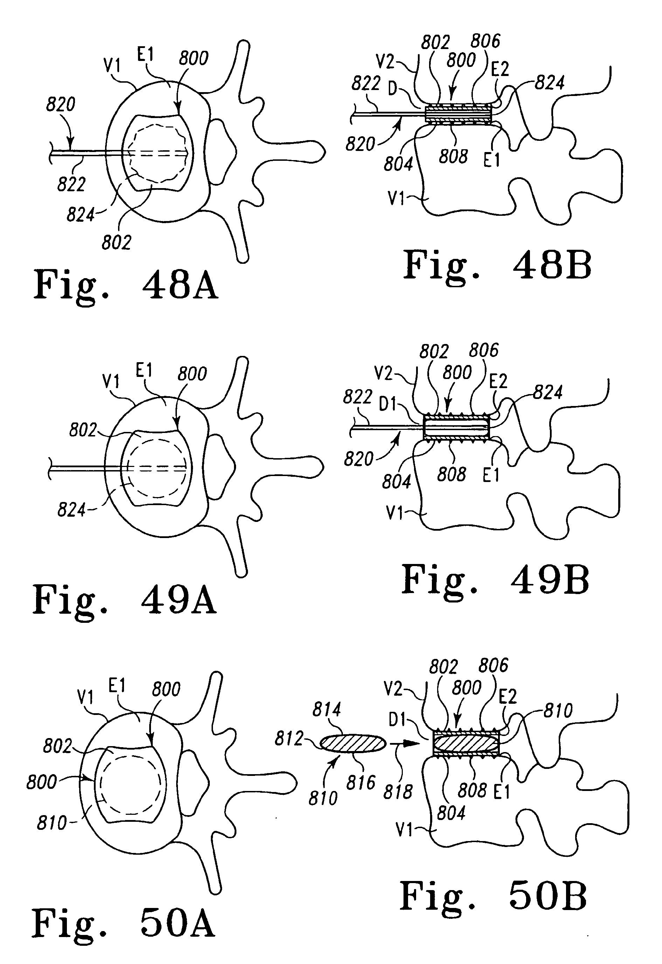

Figs. 48A and 48B are a plan view and an elevation view, respectively, of another embodiment collapsed expandable device 800 and associated delivery instrument 820 positioned in a collapsed disc space D between vertebrae V 1 and V2. The collapsed or unexpanded expandable device 800 is secured around the unexpanded expandable element 824 at a distal end of delivery instrument 800 for delivery to the collapsed disc space D in an anterior approach. Expandable device 800 includes a first portion 802 and a second portion 804 engaged along opposite sides of expandable element 824 and positionable adjacent respective ones of the endplates E1 and E2.

First and second portions 802, 804 includes a size and shape which occupies a substantial portion of the adjacent vertebral endplate to provide a large surface area for load distribution. In one embodiment, first and second portions 802, 804 occupy more than half of the vertebral endplates and include a width that extends across the spinal midline to provide bi-lateral support of the adjacent vertebrae. First and second portions 802, 804 can each include endplate contacting surfaces that are D-shaped, oval-shaped, circular, rectangular, or rectangular with rounded anterior and posterior walls as shown.

First portion 802 includes a number of engagement members 806 extending therefrom that extend toward adjacent vertebral endplate E2. Similarly, second portion 804 includes a number of engagement members 806 extending therefrom toward endplate E1. In the unexpanded condition, first and second portion 802, 804 include a height that allows engagement members 806, 808 to be moved along the endplates E1, E2 without engaging the endplates and interfering with the positioning of device 800 in the disc space.

Figs. 49A and 49B are a plan view and an elevation view, respectively, with expandable device 800 expanded by enlarging or inflating expandable element 824. In the expanded condition, first and second portions 802, 804 of expandable device 800 act on the adjacent vertebral endplates E1, E2 to distract vertebrae V1, V2 and provide a restored disc space D1. Furthermore, engagement members 806, 808 are driven into the adjacent vertebral endplates E2, E1 to achieve fixation of the first and second portions 802, 804 to vertebral V2, V1. Expandable element 824 can then be deflated and removed from expandable device 800. The relative positioning between first and second portions 802, 804 can be maintained by the engagement of the first and second portions 802, 804 with the respective vertebral endplates. It is contemplated that first and second portions 802, 804 can be interconnected with engagement members extending therebetween as discussed above, although the expansion or separation of first and second portions 802, 804 can also be maintained simply by their fixation with the respective vertebral endplates.

Figs. 50A and 50B are a plan view and an elevation view, respectively, with the expanded expandable device 800 in the restored disc space D1 of the spinal column segment and the delivery instrument 820 removed from the cavity of expandable device 800. A motion preserving device 810 can then be placed in the space or cavity between first and second portions 802, 804, as indicated by arrow 818. The motion preserving device 810 can be an elastic core as discussed above. In one embodiment, the elastic core 812 includes upper and lower convexly curved surfaces 814, 816 that contact the adjacent one of first and second portions 802, 804 to facilitate motion between the adjacent vertebra about elastic core 812.

The spinal column load can then be transferred to motion preserving device 810 to allow motion of the spinal column segment supported thereby. Transfer of the spinal column load can be accomplished by removal of a load supporting member or engagement members extending between first and second portions 802, 804, or by moving first and second portions 802, 804 toward one another, as discussed above. It is further contemplated that motion preserving device 810 can be inserted between first and second portions 802, 804 in a reduced size configuration and thereafter released or expanded to contact the adjacent first and second portions 802, 804. It is further contemplated that vertebrae V1, V2 can be over-distracted to accommodate insertion of motion preserving device 810, and then compressed to bring first and second portions 802, 804 into contact therewith.

In still another embodiment, expandable element 824 comprises an outer shell of the motion preserving device 810. In this embodiment, expandable element 824 is inflated with a suitable polymerizable material. The polymerizable material is allowed to cure in situ, and the port or shaft 822 is severed or removed so that expandable element 824 with its elastic core remaining between first and second portions 802, 804 post-operatively.

The expandable devices herein can be provided with one or more openings, windows or other structure that allows communication between the interior cavity thereof and the adjacent bony structure to facilitate bone ingrowth. The expandable devices can include a single cavity or multiple cavities. It is further contemplated that the expandable devices could be provided with support mechanisms positionable in the cavity to maintain or assist in maintaining an expanded condition of the device.

The expandable devices discussed herein can be made from any bio-compatible material, including metals, polymers and composites. Examples of metals include titanium and titanium alloys; nickel titanium alloys; stainless steel; and cobalt chrome alloys. Examples of polymers include polyaryletherketone; polyetherethereketone; polysulfone; polyolefin; polyethylene; tyrosine-based polycarbonate; polyester; polylactide; polyglicolide; polyorthoester; polyphosphazene; polyhydroxylbutyrate; and polyhydroxylvalerate, for example. Examples of composites include carbon filled composites; hydroxy-apetite filled composites; bioactive glass filled composites; and cortical bone chip filled composites, for example.

## Claims

1. A system for stabilizing a spinal column segment or for distracting a spinal disc space, comprising:
a delivery instrument (50) including a shaft (52) and an expandable element along a distal portion (54) thereof, and
an expandable device (30) including a cavity (40), the expandable device being removably mountable to the expandable element with the expandable element in the cavity and each of the expandable device and the expandable element in an unexpanded condition, wherein the expandable device is deliverable with the delivery instrument to a spinal disc space in the unexpanded condition and thereafter expandable with expansion of the expandable element to distract the spinal disc space, **characterised in that** said expandable element is a conformable expandable element (55).

2. The system of claim 1, wherein the expandable device includes a first portion (34) and a second portion (44), the first and second portions extending between distal (36) and proximal (32) ends of the expandable device.

3. The system of claim 2, wherein the first and second portions each define an outer surface with bone engagement members therealong.

4. The system of any of claims 1 to 3, further comprising:
a motion preserving device (270) positionable in the cavity.

5. The system of any preceding claim, wherein the expandable element includes a balloon structure with an interior for receiving an expansion fluid.

6. The system of claim 5, wherein the expansion fluid is selected from the group consisting of: saline solution, compressed air, and radio-contrast fluid.

7. The system of claim 5, wherein the expansion fluid is a polymerisable material.

8. The system of claim 4 or any claim dependent thereon, wherein the motion preserving device includes an elastic core (272) formed by curing the polymerisable material.

9. The system of any preceding claim, wherein the shaft of the delivery instrument includes a lumen in fluid communication with the interior of the expandable element.

10. The system of claim 2 or 3, wherein the first and second portions are movable away from one another by expanding the expandable element.

11. The system of claim 10, wherein when expanded the first and second portions define a first height adjacent the distal end of the expandable device and a second height adjacent the proximal end of the expandable device, one of the first and second heights being greater than the other of the first and second heights.

12. The system of claim 11, wherein the expandable device is tapered between the first and second heights.

13. The system of claim 1, wherein the expandable device includes a stepped configuration between the first and second heights.

14. The system of any of claims 10 to 13, wherein the first and second portions include bone growth openings (39, 49) therethrough.

15. The system of any of claims 10 to 14, wherein the first and second portions are substantially rigid and the expandable element is non-rigid.

16. The system of any of claims 10 to 15, wherein the first and second portions engage one another to maintain the expandable device in an expanded condition after removal of the expandable element from the cavity.

17. The system of claim 16, wherein at least a portion of the first and second portions is degradable to transfer load to the motion preserving device.

18. The system of any preceding claim, wherein the cavity opens at a distal end and at a proximal end of the expandable device

19. The system of any preceding claim, wherein the expandable device is radially expandable

20. The system of any preceding claim, wherein the expandable device includes a width and a height, the expandable device being expandable to increase the height while the width remains substantially constant.

21. The system of claim 1, wherein the first and second portions are structured to maintain an expanded configuration after removal of the expandable element from the cavity therebetween.

22. The system of claim 21, further comprising an elastic core positioned in the cavity.

23. The system of claim 1, further comprising bone filler material positionable in the cavity.

24. The system of claim 23, wherein the bone filler material includes bone growth promoting material.

25. The system of claim 1, wherein in the unexpanded condition the expandable device includes a banana shape.

26. The system of claim 1, wherein in an unexpanded condition the expandable device includes a D shape.

27. The system of claim 1, wherein the expandable device includes a first portion positionable along an endplate of an upper vertebra and a second portion positionable along an endplate of a lower vertebra, the first and second portions each including a size and shape to substantially occupy the adjacent endplate.

28. The system of claim 4, wherein the motion preserving device includes an elastic core having upper and lower convexly curved surfaces.

## Patentansprüche

1. System zum Stabilisieren eines Wirbelsäulensegmentes, oder zum Auseinanderhalten bzw. -ziehen eines Bandscheibenraumes mit:
einem Bereitstellungs- bzw. Abgabeinstrument (50) mit einem Schaft (52) und einem ausdehnbaren Element entlang eines distalen Abschnitts (54) hiervon; und
einer ausdehnbaren Vorrichtung (30) mit einem Hohlraum (40), wobei die ausdehnbare Vorrichtung entfernbar bzw. abnehmbar an das ausdehnbare Element anbringbar ist, wenn sich das ausdehnbare Element im Hohlraum befindet und sowohl die ausdehnbare Vorrichtung und das ausdehnbare Element in einem nicht ausgedehnten Zustand sind, wobei die ausdehnbare Vorrichtung mit dem Bereitstellungsinstrument in einen Bandscheibenraum im nicht ausgedehnten Zustand bereitstellbar und hiernach durch Ausdehnung des ausdehnbaren Elements ausdehnbar ist, um den Bandscheibenraum auseinanderzuziehen, **dadurch gekennzeichnet, dass** das ausdehnbare Element ein anpassbares ausdehnbares Element (55) ist.

2. System nach Anspruch 1, bei dem die ausdehnbare Vorrichtung einen ersten Abschnitt (34) und einen zweiten Abschnitt (44) aufweist, wobei sich der erste und der zweite Abschnitt zwischen distalen (36) und proximalen (32) Enden der ausdehnbaren Vorrichtung erstrecken.

3. System nach Anspruch 2, bei dem der erste und zweite Abschnitt jeweils eine äußere Oberfläche mit Knocheneingriffselementen entlang jener definieren.

4. System nach einem der Ansprüche 1 bis 3, ferner mit:
einer Bewegungserhaltungsvorrichtung (270), die in dem Hohlraum anordenbar ist.

5. System nach einem der vorstehenden Ansprüche, bei dem das ausdehnbare Element eine Ballonstruktur mit einem Inneren zur Aufnahme eines Ausdehnungsfluids aufweist.

6. System nach Anspruch 5, bei dem das Ausdehnungsfluid aus einer Gruppe ausgewählt ist, die besteht aus: Salzlösung, komprimierter Luft und Radiokontrastmittel.

7. System nach Anspruch 5, bei dem das Ausdehnungsfluid ein polymerisierbares Material ist.

8. System nach Anspruch 4 oder einem hiervon abhängenden Anspruch, bei dem die Bewegungserhaltungsvorrichtung einen elastischen Kern (272) aufweist, der durch Aushärten des polymerisierbaren Materials gebildet wird.

9. System nach einem der vorstehenden Ansprüche, bei dem der Schaft des Bereitstellungsinstruments ein Lumen in fluider Kommunikation mit dem Inneren des ausdehnbaren Elements aufweist.

10. System nach Anspruch 2 oder 3, bei der erste und der zweite Abschnitt voneinander weg bewegbar sind, indem das ausdehnbare Element ausgedehnt wird.

11. System nach Anspruch 10, bei dem, wenn es ausgedehnt ist, der erste und zweite Abschnitt eine erste Höhe neben dem distalen Ende der ausdehnbaren Vorrichtung und eine zweite Höhe neben dem proximalen Ende der ausdehnbaren Vorrichtung definieren, wobei eine der ersten oder zweiten Höhe größer als die andere der ersten oder zweite Höhe ist.

12. System nach Anspruch 11, bei dem die ausdehnbare Vorrichtung zwischen der ersten und zweiten Höhe verjüngt ist.

13. System nach Anspruch 1, bei dem die ausdehnbare Vorrichtung eine gestufte Konfiguration zwischen der ersten und zweiten Höhe aufweist.

14. System nach einem der Ansprüche 10 bis 13, bei dem der erste und der zweite Abschnitt Knochenwachstumsöffnungen (39, 49) hierdurch beinhalten.

15. System nach einem der Ansprüche 10 bis 14, bei dem der erste und der zweite Abschnitt im wesentlichen starr sind und das ausdehnbare Element nicht starr ist.

16. System nach einem der Ansprüche 10 bis 15, bei dem der erste und der zweite Abschnitt miteinander in Eingriff stehen, um die ausdehnbare Vorrichtung in einem ausgedehnten Zustand zu halten, nachdem das ausdehnbare Element aus dem Hohlraum entfernt wurde.

17. System nach Anspruch 16, bei dem wenigstens ein Abschnitt des ersten und zweiten Abschnitts abbaubar ist, um Last auf die Bewegungserhaltungsvorrichtung zu übertragen.

18. System nach einem der vorstehenden Ansprüche, bei dem der Hohlraum sich an einem distalen Ende und an einem proximalen Ende der ausdehnbaren Vorrichtung öffnet.

19. System nach einem der vorstehenden Ansprüche, bei dem die ausdehnbare Vorrichtung radial ausdehnbar ist.

20. System nach einem der vorstehenden Ansprüche, bei dem die ausdehnbare Vorrichtung eine Breite und eine Höhe aufweist, wobei die ausdehnbare Vorrichtung zur Vergrößerung der Höhe ausdehnbar ist, während die Breite im wesentlichen konstant bleibt.

21. System nach Anspruch 1, bei dem der erste und der zweite Abschnitt strukturiert sind, um eine ausgedehnte Konfiguration aufrechtzuerhalten, nachdem das ausdehnbare Element aus dem Hohlraum hierzwischen entfernt wurde.

22. System nach Anspruch 21, ferner mit einem elastischen Kern, der im Hohlraum angeordnet ist.

23. System nach Anspruch 1, ferner mit einem Knochenfüllermaterial, das im Hohlraum anordenbar ist.

24. System nach Anspruch 23, bei dem das Knochenfüllermaterial Knochenwachstumsförderungsmaterial aufweist.

25. System nach Anspruch 1, bei dem die ausdehnbare Vorrichtung im nichtausgedehnten Zustand eine Bananenform aufweist.

26. System nach Anspruch 1, bei dem die ausdehnbare Vorrichtung im nichtausgedehnten Zustand eine D-Form aufweist.

27. System nach Anspruch 1, bei dem die ausdehnbare Vorrichtung einen ersten Abschnitt aufweist, der entlang einer Endplatte eines oberen Wirbels positionierbar ist, und einen zweiten Abschnitt, der entlang einer Endplatte eines unteren Wirbels positionierbar ist, wobei der erste und der zweite Abschnitt jeweils eine Größe und Form beinhalten, um im wesentlichen die benachbarte Endplatte einzunehmen.

28. System nach Anspruch 4, bei dem die Bewegungserhaltungsvorrichtung einen elastischen Kern mit oberen und unteren konvex gekrümmten Oberflächen aufweist.

## Revendications

1. Système pour stabiliser un segment de colonne vertébrale ou pour distracter un espace de disque vertébral, comportant :
un instrument de distribution (50) comportant une tige (52) et un élément extensible le long d'une partie distale (54) de celui-ci ; et
un dispositif extensible (30) comportant une cavité (40), le dispositif extensible pouvant être monté de manière amovible sur l'élément extensible, l'élément extensible étant dans la cavité et chacun parmi le dispositif extensible et l'élément extensible étant dans un état non détendu, dans lequel le dispositif extensible peut être acheminé avec l'instrument de distribution vers un espace de disque vertébral à l'état non détendu et peut ensuite être détendu avec la détente de l'élément extensible pour distracter l'espace de disque vertébral, **caractérisé en ce que** ledit élément extensible est un élément extensible adaptable (55).

2. Système selon la revendication 1, dans lequel le dispositif extensible comporte une première partie (34) et une seconde partie (44), les première et seconde parties s'étendant entre des extrémités distale (36) et proximale (32) du dispositif extensible.

3. Système selon la revendication 2, dans lequel les première et seconde parties définissent chacune une surface extérieure avec des éléments de prise osseuse le long de celle-ci.

4. Système selon l'une quelconque des revendications 1 à 3, comportant également :
un dispositif de préservation de mouvement (270) pouvant être positionné dans la cavité.

5. Système selon une revendication précédente quelconque, dans lequel l'élément extensible comporte une structure à ballonnet avec un intérieur pour recevoir un fluide d'extension.

6. Système selon la revendication 5, dans lequel le fluide d'extension est sélectionné parmi le groupe constitué de :
une solution saline, de l'air comprimé et un fluide de contraste.

7. Système selon la revendication 5, dans lequel le fluide d'extension est une matière polymérisable.

8. Système selon la revendication 4 ou une revendication quelconque dépendante de celle-ci, dans lequel le dispositif de préservation de mouvement comporte un noyau élastique (272) formé en durcissant la matière polymérisable.

9. Système selon une revendication précédente quelconque, dans lequel la tige de l'instrument de distribution comporte une lumière en communication de fluide avec l'intérieur de l'élément extensible.

10. Système selon la revendication 2 ou 3, dans lequel les première et seconde parties peuvent être écartées l'une de l'autre en détendant l'élément extensible.

11. Système selon la revendication 10, dans lequel, lorsqu'elles sont détendues, les première et seconde parties définissent une première hauteur adjacente à l'extrémité distale du dispositif extensible et une seconde hauteur adjacente à l'extrémité proximale du dispositif extensible, l'une des première et seconde hauteurs étant plus grande que l'autre des première et seconde hauteurs.

12. Système selon la revendication 11, dans lequel le dispositif extensible est aminci entre les première et seconde hauteurs.

13. Système selon la revendication 1, dans lequel le dispositif extensible a une configuration étagée entre les première et seconde hauteurs.

14. Système selon l'une quelconque des revendications 10 à 13, dans lequel les première et seconde parties comportent des ouvertures de croissance osseuse (39, 49) à travers celles-ci.

15. Système selon l'une quelconque des revendications 10 à 14, dans lequel les première et seconde parties sont sensiblement rigides et l'élément extensible est non-rigide.

16. Système selon l'une quelconque des revendications 10 à 15, dans lequel les première et seconde parties coopèrent l'une avec l'autre pour maintenir le dispositif extensible dans un état détendu après le retrait de l'élément extensible de la cavité.

17. Système selon la revendication 16, dans lequel au moins une partie des première et seconde parties est dégradable pour transférer la charge au dispositif de préservation de mouvement.

18. Système selon une revendication précédente quelconque, dans lequel la cavité débouche sur une extrémité distale et sur une extrémité proximale du dispositif extensible.

19. Système selon une revendication précédente quelconque, dans lequel le dispositif extensible est radialement extensible.

20. Système selon une revendication précédente quelconque, dans lequel le dispositif extensible a une largeur et une hauteur, le dispositif extensible pouvant être détendu pour augmenter la hauteur alors que la largeur reste sensiblement constante.

21. Système selon la revendication 1, dans lequel les première et seconde parties sont structurées pour maintenir une configuration détendue après le retrait de l'élément extensible de la cavité entre celles-ci.

22. Système selon la revendication 21, comportant également un noyau élastique positionné dans la cavité.

23. Système selon la revendication 1, comportant en outre une charge osseuse pouvant être positionnée dans la cavité.

24. Système selon la revendication 23, dans lequel la charge osseuse comporte un matériau favorisant la croissance osseuse.

25. Système selon la revendication 1, dans lequel, à l'état non détendu, le dispositif extensible a une forme de banane.

26. Système selon la revendication 1, dans lequel, dans un état non détendu, le dispositif extensible a une forme de D.

27. Système selon la revendication 1, dans lequel le dispositif extensible comporte une première partie pouvant être positionnée le long d'une plaque d'extrémité d'une vertèbre supérieure et une seconde partie pouvant être positionnée le long d'une plaque d'extrémité d'une vertèbre inférieure, les première et seconde parties ayant chacune une taille et une forme pour occuper sensiblement la plaque d'extrémité adjacente.

28. Système selon la revendication 4, dans lequel le dispositif de préservation de mouvement comporte un noyau élastique ayant des surfaces supérieure et inférieure incurvées de manière convexe.
